Europäisches Patentamt

European Patent Office

Office européen des brevets

(19)

(11) Publication number: **0 115 646**
A1

(12) **EUROPEAN PATENT APPLICATION**

(21) Application number: 83200198.6

(22) Date of filing: 08.02.83

(51) Int. Cl.³: **B 01 J 23/84,** B 01 J 27/02, C 07 C 120/14, C 07 C 45/35, C 07 C 47/22

(43) Date of publication of application: 15.08.84 Bulletin 84/33

(71) Applicant: STAMICARBON B.V., Postbus 10, NL-6160 MC Geleen (NL)

(72) Inventor: Forzatti, Pio, Via Volturno, 7, Monza (IT)
Inventor: Trifiro, Ferruccio, Via Longhena, 6, Bologna (IT)
Inventor: Villa, Pierluigi, Via de Togni, 21, Milano (IT)

(74) Representative: Leherte, Georges Maurice Lucien Marie et al, Octrooibureau DSM P.O.Box 9, NL-6160 MA Geleen (NL)

(84) Designated Contracting States: **AT BE DE FR GB IT NL**

(54) Oxidation catalysts and their preparation procedure.

(57) This invention provides a catalyst composition for gas phase oxidation, ammoxidation and oxidative dehydrogenation of olefins to unsaturated aldehydes, unsaturated nitriles and dienes respectively comprising a solid solution of Fe, Sb and Ti oxides with a rutile crystal structure for at least 80% b.w., and which is represented by the empirical formula $Fe_x Sb_y Ti_z O_w$, wherein, for $x = 1$, y is in the range of 0.5 to 10, z is in the range of 0.16 to 209 and w represents the number of oxygen atoms corresponding to the oxides of the above elements. The oxide solid solution is prepared by co-precipitation of the corresponding salts followed by calcination at 500–1000 °C of the washed and dried precipitate.

EP 0 115 646 A1

1

## OXIDATION CATALYSTS AND THEIR PREPARATION PROCEDURE

This invention relates to catalysts to be used in oxidation reaction and to their preparation procedure. The catalysts according to the present invention are suitable for use in the gas phase oxidation of olefins to unsaturated aldehydes, in the gas phase ammoxidation of olefins to unsaturated nitriles, in the gas phase oxidative dehydrogenation of olefins to dienes.

The industrial developments in the last 20 years in the field of intermediates for the production of plastics and synthetic fibers have made attractive the study of processes and catalysts for the oxidation of olefins, in particular of propylene to acrolein and acrylonitrile. In view of the large capacities of the industrial plants where the above reactions are carried out, it is strongly desired to obtain conversion levels as high as possible either for economical reasons (even a slightly higher conversion results in significant additional profits) or for environmental problems (the reduction of byproducts makes easier their elmination and reduces the environmental pollution). A further very important problem relaties to the stability with use of the industrial catalysts and to their mechanical properties, particularly the resistance to abrasion, which makes possible their use in fluidized beds.

Many oxidation and ammoxidation catalysts have been described in the patent literature as the catalyst composed mainly of bismuthphosphomolybdate described in USP 2,904,580 or the catalyst composed mainly of oxides of Sb and U described in USP 3,198,750, or catalyst comprising Ce, Mo and Te oxides described in Italian Patent 682,880. Further oxidation and ammoxidation Fe and Sb oxide based catalysts are known, which are described for instance in Jap. P 420,264, in English Patent 983,755 and USP 3,197,419. It is also known that by adding particular promoters to such catalysts improved yields are obtained in the ammoxidation of propylene, as described, for example in USP 3,338,952 and USP 3,546,138 where 30 different elements are presented as suitable promoters of Fe and Sb oxide based catalysts when the amount of promoters is in the range of 1 to 10 %. The above catalysts can be used as such or supported on alumina, silica, titanium dioxide, zirconium dioxide, etc. as for instance described in English Patent 1,492,115, which claims a Fe-Sb oxide based catalyst promoted with other elements either as such or supported preferentially on silica.

All known catalysts have found not to be entirely satisfactory with respect to some characteristic such as the acrylonitrile yield or the mechanical resistance when employed in fluidized bed, the cost and the preparation procedure of the catalyst, the stability with use of catalytic activity and selectivity.

It has now been found that is is possible to prepare an active ingredient for catalyst composition with remarkable catalytic properties, good attritio resistance and which retains its initial activity even after a prolonged time on stream, starting from cheap compounds and very simple preparation procedure.

According to the present invention the active ingredient consists of a solid solution with rutile type crystal structure of Fe, Sb, and Ti oxides. For the invention, it is required that titanium oxide does interact with Fe and Sb oxides so as to give rise to a solid solution i.e. a single crystal phase. The active ingredient, according to the present invention, which consists of a solid solution of Fe, Sb, and Ti oxides with a rutile type crystal structure may be represented by the following empirical formula:

$Fe_x Sb_y Ti_z O_w$

wherein, for x = 1, y is in the range of from 0.5 to 10, z is in the range of from 0.16 to 209, and w represents the number of oxygen atoms corresponding to the oxides of the above elements. In the present invention, the preferred value of x/y is in the range of 0.2 to 1 and that of z/(x + y + z) is in the range of from 0.2 to 0.9. The active ingredient according to the present invention can be employed as such or with addition of one or more promoters to be selected from the group consisting of Mo, V, W, Bi and Te. The catalyst composition containing the promoters must contain not less than 80 % b.w. of the active ingredient. By operating according to the procedure described in this invention the promoters are substantially dissolved in the solid phase of the active ingredient with no formation of separate crystal phases.

The catalyst composition according to the present invention displays remarkable activity and stability with use in the gas phase oxidation of

3

0115646

propylene to acrolein, particularly in the ammoxidation to acrylonitrile, and in the oxydative dehydrogenation of olefins to dienes. The catalyst composition according to the present invention allows for the ammoxidation of propylene to acrylonitrile with very high yields and selectivity values even for low oxygen/propylene and ammonia/propylene ratios in a large temperature intervals. The hardness, the resistance both to abrasion and the thermal shocks of the active ingredient and of the corresponding catalyst composition allow for its use as such with no need to be supported even for operation in fluidized bed.

All the above properties are not shown by Fe and Sb oxides based catalysts either as such or in the presence of Ti oxide employed as promoter and/or support. Also in the latter case it is impossible to obtain a single phase by using a preformed $TiO_2$ although very high temperatures are employed in the calcination of this supported catalyst.

To preparre the active ingredient of this invention it is necessary to ensure an intimate contact of the different components before the calcination step during which a solid state reaction occurs bringing to a single crystal phase with a rutile type structure.

According to the present invention the oxides of Fe, Sb and Ti are preferentially prepared by coprecipitation from aqueous solutions of their salts. The soluble salts that can be used are nitrate, oxalate, chloride, and acetate in the case of Fe, pentachloride and its products of hydrolysis for Sb; tetrachloride, tetraethyltitanate, tetraisopropyltitanate and their products of alcaline hydrolysis for Ti. The coprecipitation of these salts from aqueous solutions is performed by adding volatile bases such as ammonia, aliphatic or aromatic amines with low molecular weight, and heterocyclic compounds such as pyridine, quinoline and the like, or their aqueous solutions. The temperature of coprecipitation may be in the range of from room temperature to the boiling point of the solution; however it is preferred to work at temperature in the range of from 20 to 50 °C due to the volatile bases that are used. The pH of the solution changes during coprecipitation from values below 2 up to 9. The coprecipitation is in any case completed when the pH attains permanently with time a value of 8.

A preparation procedure by coprecipitation of the mixture of oxides which is particularly convenient consists in adding a solution of anti-

4

mony chloride and titanium tetrachloride to an aqueous solution of iron chloride. Once the addition of the solution has been completed, the coprecipitation of the Fe, Sb, and Ti oxides is performed by adding an aqueous solution of from 15 % to 30 % ammonia, then by adjusting the final pH value at 8 and by keeping the temperature in the range of from 15 to 50 °C. The precipitate after separation from the solution is washed with water, preferentially with hot water, and dried at 110-130 °C. A preferred drying procedure can be performed by spray drying with warm air. The dried product is calcined at high temperature.

The calcining conditions are very important (temperature and time) because they allow for the crystalline transformation of the oxides and therefore for the activity and the other properties of the active ingredient of the catalyst composition of this invention. Although the optimum calcination conditions can vary depending on the composition of the catalyst, it is required in any case to operate at a temperature higher than 500 °C and below 1000 °C during calcination. Actually, below 500 °C the transformation of the oxides in one crystalline phase is not obtained, while temperature higher than 900 °C are not advisable for the possible variation of the morphology of the active ingredient with consequent reduction of the catalytic performances. In the preferred form of the present invention the calcination is carried out in two steps, the first characterized by a heating of the precipitate for a period of time of from 2 to 24 h at a temperature between 200 and 600 °C, the second step consisting in a further heating of the product from the first step in a range from 600 to 950 °C, maintaining this condition for a period of time of from 1 and 48 h. In the form of the invention which includes the mixing of active ingredient obtained as described with other promoting substances the moment of the addition of such substances is not critical. It is therefore possible to add the desired promotor/s to the active ingredient already calcined or to the precipitate before the calcination or in any other point of the procedure which can be considered convenient.

In the preferred form of this invention the active ingredient already calcined is impregnated with a solution of a salt, which is soluble and which is decomposed at high temperature, of the promotor/s to be added in a suitable solvent. Then the drying cycle and only the first step of calcination is repeated, because it is not necessary to repeat the high temperature calcination step, above 600 °C.

The soluble compounds for the impregnation of the calcined active ingredient which are preferred are ammonium paramolybdate, ammoniumparatungstate, ammoniummetavanadate, bismuthnitrate and telluric acid. The same components may be used also for performing the mixing of the products during the preparation of the active ingredient.

In order to illustrate better the present invention some examples of preparation, characterization and use of the active principles and of the catalytic composition are described.

## Examples

### Example 1

50 ml of anhydrous $SbCl_5$ are dissolved into 150 ml of anhydrous $TiCl_4$. The resulting solution is dropped into a solution consisting of 53.11 g of $FeCl_3$ x 6 $H_2O$ and 6.52 l of deionized water under stirring. The water volume is such that the final concentration of Fe + Sb + Ti is approximately 0.3 $\underline{M}$. Then 1100 ml of 15 % aqueous ammonia are dropped so that a final pH value of 8 is reached.

The resulting suspension is allowed to decant for 2 h. Then the precipitate is filtrated, added to 3.9 l of hot deionized water and kept at the boiling point for 5 minutes. The volume of water is such that the concentration of Fe + Sb + Ti is approximately 0.5 M. The suspension is then filtered and the washing procedure already described is repeated. After this the precipitate is dried at 120 °C, for 24 h, calcined at 350 °C for 15 h, grinded to 35-52 mesh and calcined at 900 °C for 1.5 h. The X-ray diffraction patterns correspond to that of compound No. 4 in Table 1. The product presents the empirical formula $FeSb_2Ti_{6.98}O_{19.98}$.

### Example 2

The preparation was performed as in Example 1 as far as the amounts of reagents, the coprecipitation, washing, drying and calcination (at 350 °C) procedures are concerned. The final calcination is performed at 700 °C for 3 h. Then 0.3699 g of $(NH_4)_6Mo_7O_{24}$ x 4 $H_2O$ and 0.9622 g of $H_6TeO_6$ are dissolved separately in deionized water. The two solutions are mixed and the volume of the resulting solution brought to 6.8 ml by addition of water. This solution is used to impregnate 20 g of the catalyst already prepared in a rotavapor. The modified catalyst is dried at 125 °C for 15 h and calcined by increasing the temperature (heating rate = 25 °C/30 min) up to 500 °C, where the catalyst is kept for 15 h. The X-ray diffraction patterns are very similar to those of compound No. 4 in Table 1. The product has the basic empirical formula $FeSb_2Ti_{6.98}O_{19.98}$ modified due to the addition of 0.956 % and 2.541 % b.w. of Mo and Te.

## Examples 3-6

The catalysts of examples 3-6 have been prepared with the same method of Example 2. Table 2 gives some data pertaining to the catalysts of these examples as well as of example 1.

## Examples 7-12

Active ingredients with different Ti contents have been prepared according to the procedure outlined in example 1. The products have been quoted as compound No. 2, No. 3, No. 4, No. 5, No. 6, No. 7. The amount of Ti is specified in terms of the ratio $z/(x + y + z)$ where x, y, z are numbers which refer to the empirical formula of the aforementioned compounds. In Table 1 we present the characteristic d values of the X-ray diffraction spectra of these compounds. For comparison, in Table 1, we also present the X-ray diffraction patterns of rutile $TiO_2$ (compound No. 1).

By increasing the Fe and Sb content with respect to the Ti content, a continuous shift of the X-ray 2 values is evident, which results in higher corresponding d values, thus pointing to the formation of a solid solution. Calculations performed according to standard methods of unit cell parameters do confirm that the oxides of Fe and Sb are dissolved into $TiO_2$, forming a solid solution with a rutile type structure.

## Example 13

The catalyst described in example 1, grinded to 35-52 mesh, was charged to the reactor (i.d. = 5.5 mm) electrically heated. The following gas composition (in % by volume) was fed to the reactor: $C_3H_6$, 6 %; $O_2$, 13 %; $N_2$, 81 %. The reaction temperature was changed in the range of from 322 to 472 °C; a gas hourly space velocity (GHSV) of 2700 $h^{-1}$ was employed and the pressure was set at 1.35 atm.

The results are reported hereafter. The notations are specified as follows:

X: conversion of propylene (%) = (moles of propylene reacted/moles of propylene feeded) x 100

S: selectivity to acrolein (%) = (moles of acrolein formed/moles of propylene reacted) x 100

R: yield to acrolein (%) = (moles of acrolein formed/moles of propylene feeded) x 100.

Basically only carbon oxides were detected as reaction byproducts.

| Temperature | X | S | R |
|---|---|---|---|
| 322 °C | 1.5 | 100 | 1.5 |
| 352 °C | 6. | 90 | 5.4 |
| 382 °C | 8. | 80 | 6.4 |
| 412 °C | 13. | 75 | 9.7 |
| 442 °C | 18. | 71 | 12.8 |
| 472 °C | 24. | 64 | 15.4 |

Examples 14-18

The catalysts described in examples 2, 3, 4, 5 and 6, grinded to 35-52 mesh, were charged to the reactor described in example 13. A gaseous reaction mixture with the following volumetric composition was feeded to the reactor: $C_3H_6$, 6 %; $O_2$, 13 %; $NH_3$, 7 %; $N_2$, 74 %. Other experimental conditions were: pressure, 1.5 atm, reaction temperature, variable; GHSV, slightly different depending on the catalyst. Acrylonitrile was the main product; acetonitrile, carbon oxides, HCN and acrolein were found as the main byproducts. The results, given in terms of propylene conversion (%), X, acrylonitrile selectivity (%), S, and acrylonitrile yield (%), R, are presented below. The notations are specified as follows:

X: propylene conversion (%) = (moles of propylene reacted/moles of propylene feeded) x 100

S: selectivity to acrylonitrile (%) = (moles of acrylonitrile formed/moles of propylene reacted) x 100

R: acrylonitrile yield (%) = (moles of acrylonitrile formed/moles of propylene feeded) x 100

The catalysts of examples 14-18 showed a noticeable stability with use, since no indication of both mechanical degradation and activity decay were detected after 200 h time on stream.

Catalyst No. 2 (GHSV = 520 h$^{-1}$)

| Temperature | X | S | R |
|---|---|---|---|
| 390 °C | 73 | 76 | 56 |
| 420 °C | 92 | 82 | 75 |
| 450 °C | 99 | 80 | 79 |

Catalyst No. 3 (GHSV = 520 h$^{-1}$)

| Temperature | X | S | R |
|---|---|---|---|
| 400 °C | 50 | 92 | 46 |
| 420 °C | 70 | 73 | 51 |
| 450 °C | 74 | 55 | 41 |

Catalyst No. 4 (GHSV = 510 h$^{-1}$)

| Temperature | X | S | R |
|---|---|---|---|
| 390 °C | 69 | 81 | 56 |
| 430 °C | 98 | 82 | 80 |
| 450 °C | 99 | 81 | 80 |

Catalyst No. 5 (GHSV = 510 h$^{-1}$)

| Temperature | X | S | R |
|---|---|---|---|
| 390 °C | 69 | 81 | 56 |
| 430 °C | 98 | 89 | 87 |
| 450 °C | 99 | 85 | 84 |

Catalyst No. 6 (GHSV = 640 $h^{-1}$)

| Temperature | X | S | R |
|---|---|---|---|
| 390 °C | 71 | 74 | 53 |
| 420 °C | 98 | 82 | 80 |
| 450 °C | 99 | 79 | 78 |

Table .

| TiO$_2$ | | $Fe_x Sb_y Ti_z O_w$   $x/y = 0.5$ | | | | | | | | | | | |
|---|---|---|---|---|---|---|---|---|---|---|---|---|---|
| | | $z/(x+y+z) = 0.905$ | | $z/(x+y+z) = 0.85$ | | $z/(x+y+z) = 0.7$ | | $z/(x+y+z) = 0.5$ | | $z/(x+y+z) = 0.25$ | | $z/(x+y+z) = 0.1$ | |
| compound N.1 | | compound N.2 | | compound N.3 | | compound N.4 | | compound N.5 | | compound N.6 | | compound N.7 | |
| d(Å) | I/I$_o$ | d(Å) | I/I$_o$ | d(Å) | I/I$_o$ | d(Å) | I/I$_o$ | d(Å) | I/I$_o$ | d(Å) | I/I$_o$ | d(Å) | I/I$_o$ |
| (1) | (2) | (1) | (2) | (1) | (2) | (1) | (2) | (1) | (2) | (1) | (2) | (1) | (2) |
| 3.253 | 100 | 3.248 | 100 | 3.255 | 100 | 3.255 | 100 | 3.267 | 100 | 3.263 | 100 | 3.276 | 100 |
| 2.492 | 39 | 2.494 | 53 | 2.498 | 44 | 2.509 | 61 | 2.528 | 48 | 2.537 | 52 | 2.555 | 58 |
| 2.302 | 8 | 2.298 | 8 | 2.301 | 8 | 2.303 | 18 | 2.312 | 12 | 2.309 | 13 | 2.317 | 16 |
| 2.189 | 18 | 2.190 | 21 | 2.193 | 18 | 2.202 | 20 | 2.216 | 10 | 2.223 | 7 | 2.237 | 6 |
| 2.055 | 7 | 2.053 | 8 | 2.056 | 10 | 2.059 | 12 | 2.067 | 3 | 2.062 | 5 | 2.072 | 4 |
| 1.688 | 49 | 1.689 | 62 | 1.691 | 57 | 1.697 | 58 | 1.705 | 48 | 1.708 | 46 | 1.716 | 52 |
| 1.624 | 15 | 1.624 | 20 | 1.625 | 17 | 1.629 | 20 | 1.634 | 14 | 1.633 | 13 | 1.638 | 15 |
| 1.480 | 7 | 1.487 | 10 | 1.486 | 6 | 1.498 | 21 | 1.508 | 5 | 1.522 | 5 | 1.532 | 7 |
| 1.453 | 5 | 1.453 | 10 | 1.454 | 7 | 1.457 | 16 | 1.461 | 10 | 1.462 | 11 | 1.462 | 12 |

(1) d represents the interplane spacing (in Amstrongs)

(2) I/I$_o$ represents the % ratio between the intensity of the X-ray lines, looking equal to 100 the most intense X-ray lines

Table 2

| Catalyst Example No. | empirical formula | conditions for final calcination | $z/(x+y+z)$ | elements added as promoters (% b.w.) |
|---|---|---|---|---|
| 1 | $FeSb_2Ti_{6.98}O_{19.98}$ | 900 °C, 1.5 ore | 0.7 | — |
| 2 | $FeSb_2Ti_{6.98}O_{19.98}$ | 700 °C, 3 ore | 0.7 | Mo, 0.956 %; Te, 2.541 % |
| 3 | $FeSb_2Ti_{6.98}O_{19.98}$ | 900 °C, 1,5 ore | 0.7 | Mo, 0.956 %; Te, 2.541 % |
| 4 | $FeSb_2Ti_{4.5}O_{26.4}$ | 700 °C, 3 ore | 0.6 | Mo, 1.193 %; Te, 3.172 % |
| 5 | $FeSb_{2.5}Ti_{4.5}O_{26.4}$ | 700 °C, 3 ore | 0.56 | Mo, 1.069 %; Te, 2.844 % |
| 6 | $FeSb_2Ti_{1.286}O_{8.57}$ | 700 °C, 3 ore | 0.3 | Mo, 1.752 %; Te, 4.66 % |

CLAIMS

1. Metal oxide catalyst composition comprising an active ingredient consisting of a solid solution of Fe, Sb and Ti oxides with a rutile type crystal structure and with the following empirical formula:

$Fe_xSb_yTi_zO_w$

wherein, for x = 1, y is in the range of from 0.5 to 10, z is in the range of from 0.16 to 209 and w represents the number of oxygen atoms corresponding to the oxides of the said elements.

2. A composition as claimed in claim 1 wherein 0.2  x/y  1 and 0.2  z/(x+y+z)  0.9.

3. A composition as claimed in claims 1 and 2 promoted with at least one element selected from the group consisting of Mo, V, W, Bi and Te, wherein the total amount of the promoters is in the range of from 0.2 to 20 % b.w.

4. Catalyst composition as claimed in the previous claims wherein said catalyst composition consists in at least 80 % b.w. of the solid solution with the empirical formule $Fe_xSb_yTi_zO_w$.

5. A process for preparing a catalyst composition as claimed in claim 1, wherein the active ingredient is prepared by dissolving soluble salts of Fe, Sb and Ti in an aqueous medium, by adjusting the pH of the aqueous medium to a value of about 8 by means of from 15 to 30 % aqueous ammonia, at a temperature of from 15 to 50 °C, by filtering the resulting precipitate and, after washing with water and drying at 110-130 °C, by calcining in two steps: first at a temperature of 200 to 600 °C for 2 to 24 h, then at a temperature of 500 to 1000 °C for 1 to 24 h.

6. A process as claimed in claims 3 and 5, wherein the promoter is added to the active ingredient by impregnating the said active ingredient with a solution in a proper solvent of a soluble compound of the said promoter, by drying at 110-130 °C the impregnated solid and by calcining at a temperature of 450 to 600 °C for 1 to 24 h.

7. A process as claimed in claim 6, wherein the soluble compounds of the said promoters are selected from the group consisting of ammonium paramolybdate, ammonium paratungstate, ammonium metavanadate, bismuth nitrate, and telluric acid, and wherein the solvent used in the impregnation is water.

0115646

8. A process for the gas phase oxidation of propylene to acrolein, wherein the reaction is carried out in the presence of a catalyst composition as claimed in claims 1 to 7.

9. A process for the gas phase ammoxidation of propylene to acrylonitrile wherein the reaction is carried out in the presence of a catalyst composition as claimed in claims 1 to 7.

European Patent
Office

**EUROPEAN SEARCH REPORT**

0115646

Application number

EP 83 20 0198

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | FR-A-2 202 080 (B.P. CHEM. INT. LTD.) * Page 1, line 15 - page 2, line 8; claims 1-3 * | 1-9 | B 01 J 23/84 B 01 J 27/02 C 07 C 120/14 C 07 C 45/35 C 07 C 47/22 |
| A | EP-A-0 003 158 (MONSANTO) * Abstract; pages 3-6; claims 1-10 * | 1-9 | |
| D,A | US-A-3 546 138 (J.L. CALLAHAN) * Abstract; column 1, line 25 - column 2, line 72; claims 1-4 * | 1-9 | |
| A | EP-A-0 069 439 (STANDARD OIL CO.) | | |
| A | US-A-3 625 867 (T. YOSHINO) | | **TECHNICAL FIELDS SEARCHED (Int. Cl. ³)** |
| A | US-A-3 917 682 (T. MIZUKAMI et al.) | | B 01 J C 07 C |
| A | P. PASCAL: "Nouveau traité de chimie minérale", tome IX, ed. 1963, pages 1-6,91-100, Masson et Cie, Editeurs, Paris, FR * Whole document * | | |
| D,A | GB-A-1 492 115 (NITTO CHEM. IND.) | | |

The present search report has been drawn up for all claims

| Place of search THE HAGUE | Date of completion of the search 05-10-1983 | Examiner LO CONTE C. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82